# EUROPEAN PATENT SPECIFICATION

(11) **EP 1 844 709 B1**
(45) Date of publication and mention of the grant of the patent: **11.03.2015**
(21) Application number: 06007477.0
(22) Date of filing: 10.04.2006
(51) Int. Cl.: A61B 5/107, A43D 1/02

(54) **A method and an arrangement for determining an inner sole structure adapted to a user's foot**
Verfahren und Vorrichtung zur Ermittlung der Struktur einer an dem Fuss angepassten Innensohle
Procédé et dispositif pour déterminer la structure d'une semelle intérieure adaptée au pied

(43) Date of publication of application: 17.10.2007
(73) Proprietor: currex GmbH, 20459 Hamburg (DE)
(72) Inventor: Gustafsson, Björn c/o Currex GmbH, 22085 Hamburg (DE)
(74) Representative: Eisenführ Speiser

(56) References cited:
- WO-A-03/036413
- DE-A1- 4 416 731
- JP-A- 2001 321 359
- US-A- 4 534 365
- US-A- 4 917 105
- US-A- 5 790 256

## Description

The invention relates to a method for determining an inner sole structure adapted to a user's foot. Further, an arrangement is disclosed which is adapted to determine an inner sole structure adapted to a user's foot.

The number of deformities and anatomic deviations from the regular geometry of the lower extremities is increasing. Possible explanations for this is the decreasing level of activity of humans and the increasing average body mass index. Further, inadequate or extensive sport activity may result in such diseases and deformities.

Whereas deformities of the lower extremities are treated if they severely affect the mobility of the respective person, no measures will be taken with regard to such deformities as long as they are less prominent and the person does not sense or report any impairment. However, in particular in case of sport activities it is recommendable to choose some way of support or compensation of such small deformities and an early compensation of such small deformity could stop a possible progression or avoid an increase of the deformity.

US 5,790,256 discloses a foot analyzer using pressure sensors and optical sensors to determine foot length, foot width, shoe size, foot volume, foot shape, force distribution, pronation, arch type and recommended last type. The document further discloses a method where specific data is used to provide insole selection information.

DE 44 16 731 A1 discloses a method for adapting a shoe construction to the individual contour and contact surface of a foot using a thermo-sensitive layer whereupon the shoe is placed.

US 4,534,365 discloses an apparatus comprising a temperature-responsive film whereupon the feet of a user can be placed and producing information which permits a sales person to select an appropriate foot wear or treatment to improve condition.

US 4,917,105 discloses a method for selecting an insole structure based on the plantar pressure and the angle of the foot.

JP 2001 321359 A discloses the estimation and drawing of the distribution plantar pressure from the observed static leg axis.

The invention addresses this problem and seeks to provide a method and an arrangement which allows to easily provide a tool for analysing such deformities in particular for small deformities which are not yet recognized or reported by the respective person.

To this purpose, the invention provides a method as defined in claim 1.

The method according to the invention allows an easily performed analysis of the type of foot of the user and as such an important characteristic of the lower extremity can be determined. Based on this analysis of the user's foot type a specific inner sole structure is selected as approach to compensate for certain irregular foot types or to select a structure for an irregular or neutral foot type. The inner sole structure may be comprised in a shoe or in an additional in-sole which can be inserted in regular shoes. Further, the method may be combined with a shoe system comprising a basic shoe and a number of in-soles which are selected according to step d.

The invention provides a cost-effective and precise way to analyse the foot type. The method may preferable be applied in shops when selling shoes or in-soles and can be done within short time by simply letting the customer stand on the thermosensitive sheet layer for a short period of time and analysing the thermal image of the customer's foot or feet.

The static leg axis of the user is a second important characteristic of the lower extremity. The static leg axis may easily be determined by measuring the distance between the knees of the user and the ankle joints of the user when standing upright, whereby a user having contact of the medial knee skin surfaces and the medial ankle joint skin surfaces when standing upright will have a neutral leg axis ("elongated leg"). In contrast to this, a distance between the medial knee skin surfaces will represent a bow leg axis and a distance between the medial ankle joint skin surfaces will represent a knock knees axis. The information acquired by this static leg axis analysis is important to be respected in combination with the foot type analysis. The invention is based on the important conclusion, that a flat foot type should be compensated with a neutral inner sole structure when being combined with a bow leg axis and only in case of a neutral leg axis or knock knees needs specific inner sole structures for providing stability on the medial side of the foot. In the same way, other combinations of specific foot types and leg axis are to be treated with neutral or specific inner sole structures providing lateral support, as described below.

It is particularly preferred, that the thermosensitive sheet layer comprises at least one layer which changes its colour at a certain temperature. Whereas it is basically possible to analyze the user's foot with a plurality of thermosensitive sensors arranged in one plane and connected to a computer to record the signals of each sensor and to produce an output image or result, it is preferred for the simplicity of the inventive method to analyze the foot type by directly analyzing a thermosensitive sheet layer which has changed its temperature in those regions having close contact to the user's foot. This will allow fast, cost-effective and precise analysis of the foot type, because regions of the bottom face of the foot which are not in close contact to the supporting surface will not effect an immediate colour change and thus, a differentiated image of the real contact surface of a user's foot can be directly achieved.

It is in particular preferred, that the thermosensitive sheet layer comprises a plurality of layers which change their colour at a certain temperature, whereby each layer changes its colour at a different temperature and the layers are arranged as such, that a layer changing its temperature at a higher temperature than another layer is arranged closer to the side, from which the thermosensitive sheet layer is analyzed, than the other layer. Such thermosensitive sheet layer will allow a more precise analysis because regions with high temperature (usually corresponding to high pressure) will be imaged in another way than regions of the foot with medium or low pressure (usually corresponding to medium or low pressure, respectively). The layers are arranged such, that those layers reacting at a higher temperature will overlay the layers reacting at a lower temperature, so that upon review of the image on the thermosensitive layer always the highest temperature in each region can be determined.

Thereby, the foot type is preferably classified based on the colour image of the thermosensitive layer. This will allow a person after short instruction to the method according to the invention to analyze the foot type and to determine the correct inner sole structure for the foot type.

It is further preferred, that the foot type is classified into the categories:
a. flat foot,
b. low arch foot
c. neutral foot, and/or
d. high arch foot.

Typically, acceptable results of foot type classification can be achieved by classifying the foot type in flat foot, neutral foot or high arch foot. A further precision can be achieved by additionally using a low arch foot category which is a foot type between flat foot and neutral foot. These four categories can usually easily be determined based on the temperature sensitive sheet layer changing its colour in dependency of the temperature of a user's foot.

Further it is preferred, that the leg axis type is classified into the categories:
a. bow leg
b. neutral leg, or
c. knock knees.

These three categories allow a classification of all users which have to be provided with a specific adapted inner sole structure. It is to be understood, that these categories may be further specified or divided up, e.g. in severe bow legs and slight bow legs and so on.

Further, it is preferred, that the inner sole structure type is classified in the categories
a. lateral support of the foot
b. neutral support of the foot, or
c. medial support of the foot.

These categories allow a simplified but satisfactory approach for resolving most problems associated with specific foot types or leg axis type. In particular, the lateral, neutral or medial support of the foot can be used as an effective approach for compensating irregular foot types or irregular leg axis or combinations of these.

Based on the above-referenced classification into the different foot type, leg axis and inner sole structure type categories it is specifically preferred, that the category of lateral support of the foot for the insole structure type is chosen:
a. for bow leg axis type in combination with neutral, high arch or low arch foot type, and
b. for neutral leg axis type with combination with neutral and high arch foot type,
thus achieving best results for users with such leg axis type and foot type.

Further it is preferred, that the category of neutral support of the foot for the insole structure type is chosen:
a. for bow leg axis type in combination with flat foot type,
b. for neutral leg axis type in combination low arch foot type, and
c. for knock knees leg axis type in combination neutral or high arch foot type,
and still further it is preferred, that the category of medial support of the foot for the insole structure type is chosen
a. for neutral leg axis type in combination flat foot type, and
b. for knock knees leg axis type in combination flat or low arch foot type.

The above-described selection of categories can be easily done by combining the different types of foot and leg axis with logical AND and OR operations of the categories.

According to a second aspect of the invention, a method is provided for determining an insole or a shoe adapted to a user's foot, the method comprising the steps as defined in dependent claim 11.

By this, a precise method is disclosed, which allows for fast determination of a specific shoe or insole for a user, taking into account all primarily relevant characteristics of the lower extremity of the user. It is known, to place a user's foot on a plate having an abutment for the heel of the user and a moveable abutment sliding along a scale which can be moved against a toe of the user. However, such measurement will only provide a single output and cannot take into account other dimensions than the maximum length of the user's foot. This can be improved with the method as described above, because the data acquired by the thermosensitive sheet layer represents the foot size in each direction and further represents this foot size with additional information as to the foot pressure in each region under the sole.

The problem underlying the invention is further solved with an arrangement for determining an inner sole structure adapted to a user's feet, as defined in independent claim 12.

The arrangement may preferably be used to perform the method according to the invention. The logical operator may be represented by a written instruction listing of the different logical possibilities or a software solution having implemented such logical operations for calculating the inner sole type category after inputting the user's foot type.

The arrangement includes a look-up table comprising
- a first field with at least two foot type categories,
- a second field with at least two leg axis type categories
wherein the logical operator is adapted to define at least two inner sole type categories depending on the foot type category and the leg axis type category. The look-up table allows a user to determine the inner sole type category in a fast and precise way. The look-up table may be integrated in a software or a computer system or a small computer hand-held or the like. Further, the look-up table can be a written documentation or paper listing the different combinations and results of foot type and leg axis type and the inner sole structure types for each combination of the former two types.

It is further preferred, that the thermosensitive sheet layer comprises at least one, preferably several layers which change their colour in response to a certain temperature level, whereby the temperature levels are adapted to the temperature range of a user's feet. The inventor has recognised, that by this a cheap way to analyze pressure under the user's foot is provided because the temperature transferred by the foot usually corresponds to the sole pressure. Further mistakes in the analysis by the user are avoided by the simplicity of the method.

In particular, it is preferred, that at least one length scale is arranged beside the thermosensitive layer for determining the user's foot size. By this, the foot size of the user can easily be determined in the same step of the analysis of the foot temperature, i.e. the foot sole pressure. In particular, it is possible to gain a number of geometric parameters from the image of the foot on the thermosensitive layer sheet and these geometric properties could be determined with e.g. two length scales arranged perpendicular to each other beside the thermosensitive layer.

The above-described arrangements comprising a look-up table may be further improved in that the look-up table is a print-out and the categories in the fields are represented by different symbols. This allows easy handling of the arrangement according to the invention.

Further, it is preferred that in the look-up table
a. each foot type category is represented by a different colour,
b. each leg axis category is represented by a different geometric symbol, and
c. each inner sole structure type is represented by a coloured geometric symbol, the colour of which corresponding to the respective foot type category and the geometric symbol corresponding to the respective leg axis category which combination is determined by the logical operator to correspond to the respective inner sole structure type category.

By this, the look-up table can be handled by persons after short instruction to the inventive arrangement and mistakes in the analysis using the inventive arrangement can be avoided. The combination of colour and geometric symbol to a result of a coloured geometric symbol allows for a number of categories for foot type and leg axis. Certainly, the assignment can be reversed, i.e. the foot type can be represented by a geometric symbol and the leg axis can be represented by a different colour.

In particular, it is preferred, that the look-up table comprises a third field with at least two inner sole structure type categories. This will speed up diagnosis with the arrangement according to the invention.

Finally, the look-up table according to the invention can be further improved in that
a. the first field comprises a flat foot, low arch foot, neutral foot and high arch foot category,
b. the second field comprises a bow leg, neutral leg and knock knees category, and/or
c. the third field comprises a lateral, neutral and medial foot support category.

To this extent and to the logical operations for calculating and determining a certain inner sole structure type from the foot type and the leg axis type it is referred to the above described method according to the invention and it is to be understood, that the arrangement according to the invention may preferably comprise a logical implementation of these combinations.

According to another aspect of the invention, the method according to the invention may be implemented in a computer program product, which is adapted to perform the following steps when being exercised on a computer:
a. receiving a foot type category as a first input,
b. receiving a leg axis type category as a second input,
c. determining an inner sole structure type category based on the first and second input, in particular using logical operations according to claims 9 through 11, and
d. outputting the inner sole structure type category determined in step c).

The computer program product may be further improved in that step a) comprises
- receiving foot type data acquired by thermal analysis of a bottom face of a user's foot,
- analyzing the foot type data, and
- determining the foot type of the user based on the data using image processing.

A preferred embodiment of the invention is described with reference to the figures, wherein:
Fig. 1 shows a perspective view of an analyzing device for a foot type, and
Fig. 2 shows an exemplary look-up table according to the invention.

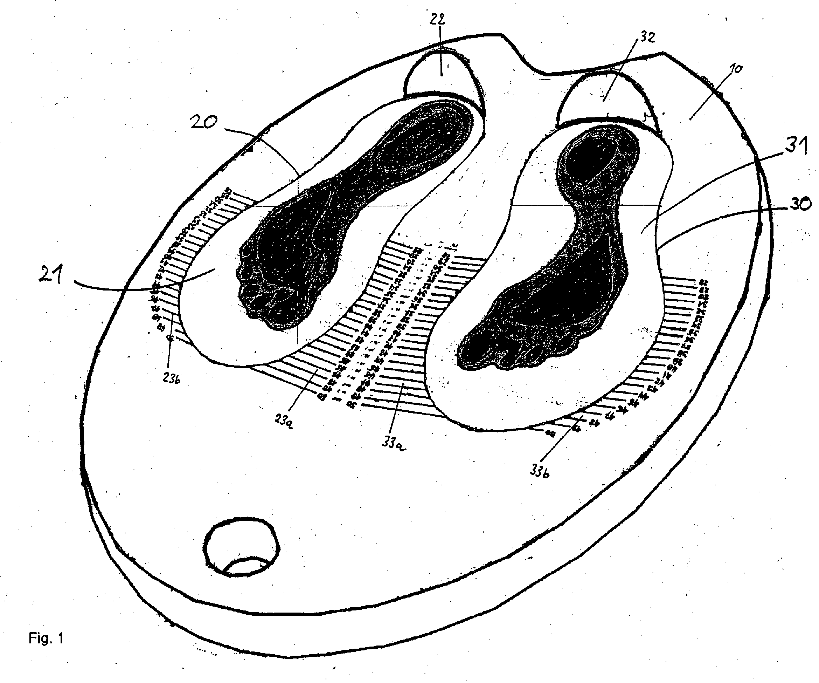

Referring to Fig. 1, the analyzing device comprises a plate 10 having two recesses 20,30 on the upper side, each recess being formed in the contour of a shoe but being dimensioned larger than regular shoe sizes.

A thermosensitive layer 21,31 is secured within the recesses 20,30. As can be seen in Fig. 1, the thermosensitive layer 21,31 is reactive to the temperature of the user's foot and changes its colour depending on the local temperature distribution. A typical imprint of the left and right foot of the user is shown onto the thermosensitive layers 21,31 in Fig. 1.

At the side where the heel of the user is arranged when standing on the thermosensitive layer 21,31, a heel abutment cap 22,32 with a rounded contour is fixed to the plate 10. The user is instructed to place his feet onto the thermosensitive layers 21,31 with his heels abutting the heel abutment caps 22,32.

A scale 23a,33a is arranged on the medial side of the thermosensitive layers 21,31 and comprises a plurality of lines arranged parallel to each other and perpendicular to the length axis of the user's foot when placing it on the thermosensitive layer 21,31. The scale is arranged in the toe region and allows easy determination of the length of the user's foot. The scale 23a,33a further comprises shoe size numbers corresponding to each respective of the parallel lines of the scale.

A further scale 23b,33b is arranged on the lateral side of the thermosensitive layer and allows identical determination of the shoe size. Scales 23a,33a, 23b,33b are preferably divided in European shoe sizes. The shoe size determined by this may be calculated in the corresponding shoe size of a different classification (e.g. French stitch or UK sizes) using a table of concordance included in the look-up table.

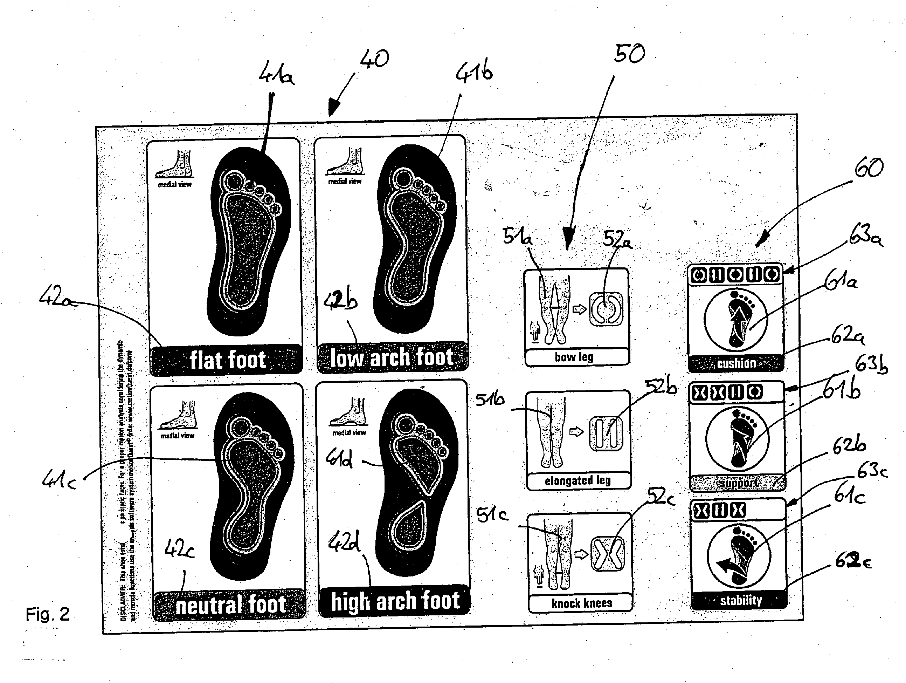

Fig. 2 shows an exemplary look-up table according to the invention. In a first field 40, a number of four foot type categories 41a-d is listed. Each category comprises an image 41 and with the typical result of the analysis of the particular foot type with the thermosensitive layer. Further, the foot type is designated in written in a lower section 42a-d of each category and this lower section is kept in a specific colour. By this, the sector 42a designating "flat foot", the sector "low arch foot", the sector 42c designating "neutral foot" and the sector 42d designating "high arch foot" are kept in different colours, so that a total of four colours represent the four different categories of foot type.

A second field 50 lists a total number of three different leg axis types. Each category in this field comprises an image representing the type of leg axis 51a-c and a symbol 52a-c for the specific category. By this, the category "bow leg", "elongated leg" and "knock knees" is represented by symbols 52a-c, so that a total of three symbols sufficient for describing the leg axis type of the user.

Finally, the look-up table comprises a third field 60 having three categories of inner sole structure types. Each category comprises an image 61a-c which is represented for the type of support which is provided by the specific inner sole structure. Further, in a lower section of each category, a written description of the type of inner sole structure and a specific colour 62a-c is included. This written description and/or colour may be used to mark the in-soles, the shoes and/or the packages of the in-soles of the inside of the shoes comprising the specific inner sole structure.

Further, in an upper section 62a-c, a number of coloured symbols is included in each category. The symbols in this upper section 62a-c correspond to the symbols 52a-c and the colours of the symbols in upper section 62a-c correspond to the colours 42a-c. By this, the user can find each combination of any colour 42a-d and any symbol 52a-c in one of the upper sections 63a-c. By this, each combination of any colour 42a-d and any symbol 52a-c is logically connected with a certain inner sole structure type category in the third field 60. This allows easy determination of the right inner sole structure for a user having a specific foot type and a specific leg axis.

The method according to the invention will be conducted as follows:

First, the user places his feet barefoot or with the socks on onto the thermosensitive layer 21,31 of the plate 10 with the heels striking against the heel abutment caps 22,32. The user rests for 5 through 15 seconds on the thermosensitive layer and then removes his feet from the layers.

After this, the length of the left and right foot is determined based on the thermal imprint using scales 33a,b and 23a,b. Further, the foot type is analyzed on the basis of the thermal imprint shown by the thermosensitive layer.

Now, the user is asked to stand upright and the distance between the medial faces of the knees and of the ankle joints is measured, e.g. by introducing one or two fingers between the knees and between the ankle joints.

Depending on the image of the thermosensitive layers, a type of foot according to one of the categories in the first field is determined and the colour 42a-d is used for further processing. Depending on the type of leg axis, one of the three categories in the second field 50 is determined and the symbol 52a-c of this category is used for further processing.

Finally, the user has simply to look for the respective symbol in the respective colour in the third field 60 to determine the type of inner sole structure which should be used for his feet. The symbols of the third field 60 may be represented on the packages of the corresponding shoes or insoles or the colours 62a-c may be used for such marking of the corresponding insoles or shoes.

## Claims

1. A method for determining an inner sole structure adapted to a user's feet, the method comprising the steps:
a. placing the plantar sole of one foot or both feet of the user on a thermosensitive sheet layer,
b. analysing the imprint on the thermosensitive sheet layer, in particular analysing the contact surface of the longitudinal arch on the imprint,
c. classifying the type of foot of the user in at least two foot type categories based on the imprint,
d. determining an inner sole structure type based on the foot type category determined in step c),
**characterized by** the steps:
e. determining the static leg axis of the user by measuring the distance between the knees of the user and the ankle joints of the user when standing upright wherein a user having
- contact of the medial knee skin surfaces and the medial ankle joint skin surfaces when standing upright will have a neutral leg axis,
- a distance between the medial knee skin surfaces will represent a bow leg axis,
- a distance between the medial ankle joint skin surfaces will represent a knock knees axis,
f. classifying the type of leg axis of the user in at least three leg axis categories, wherein determining an inner sole structure type is based on the foot type category determined in step c) and the leg axis category determined in step f).

2. The method according to claim 1,
wherein the thermosensitive sheet layer comprises at least one layer which changes its colour at a certain temperature.

3. The method according to claim 2,
wherein the thermosensitive sheet layer comprises a plurality of layers which change their colour at a certain temperature, whereby each layer changes its colour at a different temperature and the layers are arranged as such, that a layer changing its temperature at a higher temperature than another layer is arranged closer to the side, from which the thermosensitive sheet layer is analyzed, than the other layer.

4. The method according any of the preceding claims 2-3,
wherein the foot type is classified based on the colour image of the thermosensitive layer.

5. The method according any of the preceding claims,
wherein the foot type is classified into the categories:
a. flat foot,
b. neutral foot,
c. high arch foot, and/or
d. low arch foot.

6. The method according any of the preceding claims,
wherein the leg axis type is classified into the categories:
a. bow leg
b. neutral leg, or
c. knock knees.

7. The method according any of the preceding claims,
wherein the inner sole structure type Is classified in the categories
a. lateral support of the foot
b. neutral support of the foot, or
c. medial support of the foot.

8. The method according to the preceding claims 5-7,
wherein the category of lateral support of the foot for the insole structure type is chosen:
a. for bow leg axis type in combination with neutral, high arch or low arch foot type, and
b. for neutral leg axis type in combination with neutral and high arch foot type.

9. The method according the preceding claims 5-7 or claim 8,
wherein the category of neutral support of the foot for the insole structure type is chosen:
a. for bow leg axis type in combination with flat foot type.
b. for neutral leg axis type in combination with low arch foot type, and
c. for knock knees leg axis type in combination with neutral or high arch foot type.

10. The method according to the preceding claims 5-7 or claim 8 or 9,
wherein the categories medial support of the foot for the insole structure type is chosen
a. for neutral leg axis type in combination with flat foot type, and
b. for knock knees leg axis type in combination with flat or low arch foot type.

11. A method for determining an insole or a shoe adapted to a user's feet, the method comprising the steps,
a. determining the inner sole structure according to any of the preceding claims, and
b. determining the foot size based on the data acquired by the thermosensitive sheet layer.

12. Arrangement for determining an inner sole structure adapted to a user's feet, comprising:
a. a thermosensitive sheet layer (21, 31) adapted to receive the bottom face of the user's foot or feet,
b. a logical operator (60) defining at least two inner sole type categories depending on the foot type,
**characterized by**
c. a look-up table comprising
- a first field (40) with at least two foot type categories,
- a second field (50) with at least three leg axis type categories
wherein the logical operator is adapted to define at least two inner sole type categories depending on the foot type and the leg axis type category, said leg axis being defined by the distance between the knees of the user and the ankle joints of the user when standing upright, wherein a user having contact of the medial knee skin surfaces and the medial ankle joint skin surfaces when standing upright is defined as having a neutral leg axis, a distance between the medial knee skin surfaces defines a bow leg axis and a distance between the medial ankle joint skin surfaces defines a knock knees axis.

13. Arrangement according to claim 12,
wherein the thermosensitive sheet layer comprises at least one, preferably several layers which change their colour in response to a certain temperature level, whereby the temperature levels are adapted to the temperature range of a user's feet.

14. Arrangement according to claim 12 or 13,
wherein at least one length scale is arranged beside the thermosensitive layer for determining the user's foot size.

15. Arrangement according to any of the preceding claims 12 through 14,
wherein the look-up table is a print-out and the categories in the fields are represented by different symbols.

16. Arrangement according to any of the preceding claims 12 through 15, wherein
a. each foot type category is represented by a different colour,
b. each leg axis category is represented by a different geometric symbol, and
c. each inner sole structure type is represented by a coloured geometric symbol, the colour of which corresponding to the respective foot type category and the geometric symbol corresponding to the respective leg axis category which combination is combined by the logical operator to the respective inner sole structure type.

17. Arrangement according to any of the preceding claims 12 through 16,
wherein the look-up table comprises a third field (60) with at least two inner sole structure type categories.

18. Arrangement according to any of the preceding claims 12 through 17, wherein
a. the first field (40) comprises a flat foot, low arch foot, neutral foot and high arch foot category,
b. the second field (50) comprises a bow leg, neutral leg and knock knees category, and/or
c. the third field (60) comprises a lateral, neutral and medial foot support category.

19. A computer program product, which is adapted to perform the following steps when being exercised on a computer:
a. receiving a foot type category as a first input,
b. receiving a leg axis type category as a second input, said leg axis being defined by the distance between the knees of the user and the ankle joints of the user when standing upright, wherein a user having contact of the medial knee skin surfaces and the medial ankle joint skin surfaces when standing upright is defined as having a neutral leg axis, a distance between the medial knee skin surfaces defines a bow leg axis and a distance between the medial ankle joint skin surfaces defines a knock knees axis.
c. determining an inner sole structure type category based on the first and second input, using logical operations according to previous claims 8 through 10, and
d. outputting the inner sole structure type category determined in step c).

20. A computer program product according to claim 19,
wherein step a) comprises
- receiving foot type data acquired by thermal analysis of a bottom face of a user's foot,
- analyzing the foot type data, and
- determining the foot type of the user based on the data using image processing.

## Patentansprüche

1. Eine Methode zur Ermittlung einer Innensohlenkonstruktion angepasst an des Nutzers Füße, die Methode umfassend folgende Schritte:
a. Platzieren der plantaren Sohle eines oder beider Füße des Nutzers auf einer temperatursensitiven Folien-Lage,
b. Analysieren des Abdrucks auf der temperatursensitiven Folien-Lage, insbesondere analysieren der kontaktierten Oberfläche des längsverlaufenden Fußgewölbes auf dem Abdruck,
c. Klassifizieren des Fußtyps des Nutzers in mindestens zwei Fußtypen-Kategorien basierend auf dem Abdruck,
d. Ermitteln eines Innensohlenkonstruktions-Typ basierend auf der ermittelten Fußtypen-Kategorie in Schritt c),
**gekennzeichnet durch** die Schritte:
e. Ermitteln der statischen Beinachse des Nutzers **durch** messen des Abstands zwischen den Knien des Nutzers und den Fußgelenken des Nutzers, wenn dieser aufrecht steht, wobei ein Nutzer,
- der Kontakt hat zwischen den medialen Kniehautoberflächen und den medialen Fußgelenkhautoberflächen, über neutrale Beinachsen verfügt,
- der einen Abstand zwischen den medialen Kniehautoberflächen hat, 0-Beine hat,
- der einen Abstand zwischen den medialen Fußgelenkhautoberflächen hat, X-Beine hat,
f. Klassifizieren des Typs der Beinachse des Nutzers in mindestens drei Beinachsen-Kategorien, worin die Ermittlung eines Innensohlenkonstruktionstyp basiert auf der Fußtyp-Kategorie, ermittelt in Schritt c) und der Beinachsen-Kategorie, ermittelt in Schritt f).

2. Methode nach Anspruch 1, worin die temperatursensitive Folien-Lage wenigstens eine Lage umfasst, welche ihre Farbe bei einer Grenztemperatur wechselt.

3. Methode nach Anspruch 2, worin die temperatursensitive Folien-Lage eine Mehrzahl von Lagen umfasst, welche ihre Farbe bei einer bestimmten Temperatur ändern, wobei jede Lage ihre Farbe bei einer anderen Temperatur wechselt und die Lagen so angeordnet sind, dass eine Lage die ihre Farbe bei einer höheren Temperatur ändert als eine andere Lage näher zu der Seite angeordnet ist auf welcher die temperatursensitive Folien-Lage analysiert wird, als die andere Lage.

4. Methode nach einem der vorhergehenden Ansprüche 2 bis 3, worin der Fußtyp basierend auf dem farblichen Abbild der temperatursensitiven Lagen klassifiziert wird.

5. Methode nach einem der vorhergehenden Ansprüche, worin der Fußtyp klassifiziert wird in die Kategorien:
a. Plattfuß,
b. Neutralfuß,
c. Hochwölbfuß, und/oder
d. Niedrigwölbfuß.

6. Methode nach einem der vorhergehenden Ansprüche, worin der Beinachsen-Typ klassifiziert wird in die Kategorien:
a. O-Beine,
b. Neutral-Beine, oder
c. X-Beine.

7. Methode nach einem der vorhergehenden Ansprüche, worin die Innensohlenkonstruktion klassifiziert wird in die Kategorien:
a. seitliche Unterstützung des Fußes,
b. neutrale Unterstützung des Fußes, oder
c. mediale Unterstützung des Fußes.

8. Methode nach einem der vorhergehenden Ansprüche 5 bis 7, worin die Kategorie seitliche Unterstützung des Fußes für den Einlagen-Struktur-Typ ausgewählt wird:
a. für O-beinigen Beinachsen-Typ in Kombination mit neutralem, hoch gewölbtem oder niedrig gewölbtem Fuß, und
b. für neutralen Beinachsen-Typ in Kombination mit neutralem und hochgewölbtem Fußtyp.

9. Methode nach einem der vorhergehenden Ansprüche 5 bis 8, worin die Kategorie neutrale Unterstützung des Fußes für den Einlagen-Struktur-Typ ausgewählt wird:
a. für O-beinigen Beinachsen-Typ in Kombination mit flachem Fußtyp,
b. für neutralen Beinachsen-Typ in Kombination mit niedrig gewölbten Fußtyp, und
c. für X-beinigen Beinachsen-Typ in Kombination mit neutralem, oder hoch gewölbtem Fußtyp.

10. Methode nach einem der vorhergehenden Ansprüche 5 bis 9, worin die Kategorie mediale Unterstützung des Fußes für den Einlagen-Struktur-Typ ausgewählt wird:
a. für neutralen Beinachsen-Typ in Kombination mit flachem Fußtyp, und
b. für X-beinigen Beinachsen-Typ in Kombination mit flachem, oder niedrig gewölbtem Fußtyp.

11. Methode zur Ermittlung einer Einlage oder Schuhes, angepasst an die Füße des Nutzers, die Methode umfasst die Schritte,
a. Ermittlung der Innensohlenkonstruktion gemäß einem der vorhergehendenden Ansprüche, und
b. Ermittlung der Fußgröße basierend auf den ermittelten Daten von dem temperatursensitiven Folien-Lage.

12. Anordnung zur Ermittlung einer Innensohlenkonstruktion angepasst an die Füße eines Nutzers, umfassend:
a. eine temperatursensitive Folien-Lage (21, 31) angepasst zum Aufnehmen der Unterseite des Fußes oder der Füße des Nutzers,
b. ein logischer Operator (60) der mindestens zwei Innensohlenkonstruktions-Kategorien definiert abhängig vom Fußtyp,
charakterisiert durch
c. eine Nachschlagetabelle umfassend
- ein erstes Feld (40) mit mindestens zwei Fußtyp-Kategorien,
- ein zweites Feld (50) mit mindestens drei Beinachsen-Typ-Kategorien,
worin der logische Operator eingesetzt ist zum Definieren von mindestens zwei Innensohlenkonstruktionen abhängig von dem Fußtyp und der Beinachsen-Typ-Kategorie, wobei die Beinachse definiert ist als der Abstand zwischen den Knien des Nutzers und den Fußgelenken des Nutzers, wenn dieser aufrecht steht, wobei ein Nutzer,
- der Kontakt hat zwischen den medialen Kniehautoberflächen und den medialen Fußgelenkhautoberflächen, über neutrale Beinachsen verfügt,
- der einen Abstand zwischen den medialen Kniehautoberflächen hat, O-Beine hat,
- der einen Abstand zwischen den medialen Fußgelenkhautoberflächen hat, X-Beine hat,

13. Anordnung nach Anspruch 12, worin die temperatursensitive Folien-Lage mindestens eine, vorzugsweise mehrere Lagen umfasst, welche ihre Farbe in Abhängigkeit von einem bestimmten Temperaturniveau ändern, wobei die Temperaturniveaus auf den Temperaturbereich der Füße des Nutzers abgestimmt sind.

14. Anordnung nach Anspruch 12 oder 13, worin mindestens eine Längenskala seitlich der temperatursensitiven Folien-Lage angeordnet ist, zur Ermittlung der Fußgröße des Nutzers.

15. Anordnung nach einem der vorhergehendenden Ansprüche 12 bis 14, worin die Nachschlagetabelle ausgedruckt ist und die Kategorien in den Feldern durch verschiedene Symbole dargestellt sind.

16. Anordnung nach einem der vorhergehendenden Ansprüche 12 bis 15, worin
a. jede Fußtyp-Kategorie durch eine unterschiedliche Farbe dargestellt wird,
b. jede Beinachsen-Kategorie ist durch ein unterschiedliches geometrisches Symbol dargestellt, und
c. jeder Innensohlenkonstruktionstyp ist dargestellt durch ein farbiges geometrisches Symbol, dessen Farbe mit der entsprechenden Fußtyp-Kategorie korrespondiert und das geometrische Symbol mit der entsprechenden Beinachsen-Kategorie korrespondiert, wobei deren Kombination durch den logischen Operator zu einen Innensohlenkonstruktlonstyp kombiniert ist.

17. Anordnung nach einem der vorhergehendenden Ansprüche 12 bis 16, worin die Nachschlagetabelle ein drittes Feld (60) enthält, mit mindestens zwei Innensohlenkonstruktionstyp-Kategorien.

18. Anordnung nach einem der vorhergehendenden Ansprüche 12 bis 17, worin
a. das erste Feld (40) eine Plattfuß-, Niedrlgwölbfuß-, Neutralfuß- und Hochwälbfuß-Kategorie,
b. das zweite Feld (50) eine O-Bein-, Neutral-Bein-, X-Bein-Kategorie beinhaltet und/oder
c. das dritte Feld (60) eine seitliche, neutrale oder mediale Fuß-Unterstützungs-Kategorie beinhaltet.

19. Computerprogramm-Produkt, welches zum Ausführen der folgenden Schritte bei Ausführung auf einem Computer ausgebildet ist:
a. Empfangen einer Fußtyp-Kategorie als eine erste Eingabe,
b. Empfangen einer Beinachsen-Typ-Kategorie als eine zweite Eingabe, wobei die Beinachse definiert ist als der Abstand zwischen den Knien des Nutzers und den Fußgelenken des Nutzers wenn dieser aufrecht steht, wobei ein Nutzer,
- der Kontakt hat zwischen den medialen Kniehautobertlächen und den medialen Fußgelenkhautoberflächen, über neutrale Beinachsen verfügt,
- der einen Abstand zwischen den medialen Kniehautoberflächen hat, O-Beine hat,
- der einen Abstand zwischen den medialen Fußgelenkhautoberflächen hat, X-Beine hat.
c. Ermitteln einer Innensohlenkonstruktionstyp-Kategorie basierend auf der ersten und zweiten Eingabe, unter Anwenden der logischen Operationsbedingungen nach den vorangegangenen Ansprüchen 8 bis 10 und
d. Ausgeben der im Schritt c) ermittelten Innensohlenkonstruktionstyp Kategorie.

20. Computerprogramm Produkt nach Anspruch 19, worin der Schritt a) umfasst:
- Empfangen der Fußtyp-Daten erfasst von der Temperatur-Auswertung einer Unterfläche eines Fußes eines Nutzers,
- Analysieren der Fußtyp-Daten, und
- Ermitteln des Fußtyps des Nutzers basierend auf den Daten unter Einsatz einer Bildverarbeitung.

## Revendications

1. Procédé de détermination d'une structure de semelle intérieure adaptée aux pieds d'un utilisateur, le procédé comprenant les étapes de :
a. placement de la semelle plantaire d'un pied ou des deux pieds de l'utilisateur sur une couche de feuille thermosensible,
b. analyse de l'empreinte sur la couche de feuille thermosensible, en particulier analyse de la surface de contact de la voûte longitudinale sur l'empreinte,
c. classification du type de pied de l'utilisateur dans au moins deux catégories de type de pied basées sur l'empreinte,
d. détermination d'un type de structure de semelle intérieure basé sur la catégorie de type de pied déterminée à l'étape c),
**caractérisé par** les étapes de :
e. détermination de l'axe de jambe statique de l'utilisateur en mesurant la distance entre les genoux de l'utilisateur et les articulations de cheville de l'utilisateur lorsqu'il se tient debout dans lequel un utilisateur ayant
- un contact des surfaces de peau internes du genou et des surfaces de peau internes de l'articulation de la cheville lorsqu'il se tient debout aura un axe de jambe neutre,
- une distance entre les surfaces de peau internes du genou représentera un axe de jambe arquée,
- une distance entre les surfaces de peau internes de l'articulation de la cheville représentera un axe de genoux cagneux,
f. classification du type d'axe de jambe de l'utilisateur en au moins trois catégories d'axe de jambe, dans lequel la détermination d'un type de structure de semelle intérieure est basée sur la catégorie de type de pied déterminée à l'étape c) et la catégorie d'axe de jambe déterminée à l'étape f).

2. Procédé selon la revendication 1,
dans lequel la couche de feuille thermosensible comprend au moins une couche qui change de couleur à une certaine température.

3. Procédé selon la revendication 2,
dans lequel la couche de feuille thermosensible comprend une pluralité de couches qui changent de couleur à une certaine température, moyennant quoi chaque couche change de couleur à une température différente et les couches sont agencées de sorte qu'une couche changeant de couleur à une température plus élevée qu'une autre couche est agencée plus près du côté, à partir duquel la couche de feuille thermosensible est analysée, que l'autre couche.

4. Procédé selon l'une quelconque des revendications 2 à 3 précédentes,
dans lequel le type de pied est classé en se basant sur l'image couleur de la couche thermosensible.

5. Procédé selon l'une quelconque des revendications précédentes,
dans lequel le type de pied est classé en les catégories suivantes :
a. pied plat,
b. pied neutre,
c. pied à voûte plantaire très cambrée, et/ou
d. pied à voûte plantaire faiblement cambrée.

6. Procédé selon l'une quelconque des revendications précédentes,
dans lequel le type d'axe de jambe est classé en les catégories suivantes :
a. jambe arquée,
b. jambe neutre, ou
c. genoux cagneux.

7. Procédé selon l'une quelconque des revendications précédentes,
dans lequel le type de structure de semelle intérieure est classé en les catégories suivantes :
a. support latéral du pied,
b. support neutre du pied, ou
c. support interne du pied.

8. Procédé selon les revendications 5 à 7 précédentes,
dans lequel la catégorie de support latéral du pied pour le type de structure de semelle intérieure est choisie :
a. pour un type d'axe de jambe arquée en combinaison avec un type de pied à voûte neutre, voûte plantaire très cambrée ou voûte plantaire faiblement cambrée, et
b. pour un type d'axe de jambe neutre en combinaison avec un type de pied à voûte plantaire neutre et à voûte plantaire très cambrée.

9. Procédé selon les revendications 5 à 7 ou la revendication 8 précédentes,
dans lequel la catégorie de support neutre du pied pour le type de structure de semelle intérieure est choisie :
a. pour un type d'axe de jambe arquée en combinaison avec un type de pied plat,
b. pour un type d'axe de jambe neutre en combinaison avec un type de pied à voûte plantaire faiblement cambrée, et
c. pour un type d'axe de jambe à genoux cagneux en combinaison avec un type de pied à voûte plantaire neutre ou très cambrée.

10. Procédé selon les revendications 5 à 7 ou la revendication 8 ou 9 précédentes, dans lequel la catégorie support interne du pied pour le type de structure de semelle intérieure est choisie
a. pour un type d'axe de jambe neutre en combinaison avec un type de pied plat, et
b. pour un type d'axe de jambe à genoux cagneux en combinaison avec un type de pied plat ou à voûte plantaire faiblement cambrée.

11. Procédé de détermination d'une semelle intérieure ou d'une chaussure adaptée aux pieds d'un utilisateur, le procédé comprenant les étapes de
a. détermination de la structure de semelle intérieure selon l'une quelconque des revendications précédentes, et
b. détermination de la pointure du pied en se basant sur les données acquises par la couche de feuille thermosensible.

12. Agencement permettant de déterminer une structure de semelle intérieure adaptée aux pieds d'un utilisateur, comprenant :
a. une couche de feuille thermosensible (21, 31) adaptée pour recevoir la face de dessous du pied ou des pieds d'un utilisateur,
b. un opérateur logique (60) définissant au moins deux catégories de type de semelle intérieure en fonction du type de pied,
**caractérisé par**
c. une table de consultation comprenant
- un premier champ (40) avec au moins deux catégories de type de pied,
- un deuxième champ (50) avec au moins trois catégories de type d'axe de jambe dans lequel l'opérateur logique est adapté pour définir au moins deux catégories de type de semelle intérieure en fonction du type de pied et de la catégorie de type d'axe de jambe, ledit axe de jambe étant défini par la distance entre les genoux de l'utilisateur et les articulations de cheville de l'utilisateur lorsqu'il se tient debout, dans lequel un utilisateur ayant un contact des surfaces de peau internes du genou et des surfaces de peau internes de l'articulation de la cheville lorsqu'il se tient debout est défini comme ayant un axe de jambe neutre, une distance entre les surfaces de peau internes du genou définit un axe de jambe arquée, et une distance entre les surfaces de peau internes de l'articulation de cheville définit un axe de genoux cagneux.

13. Agencement selon la revendication 12,
dans lequel la couche de feuille thermosensible comprend au moins une, de préférence plusieurs couches qui changent de couleur en réponse à un certain niveau de température, moyennant quoi les niveaux de température sont adaptés à la plage de températures des pieds d'un utilisateur.

14. Agencement selon la revendication 12 ou 13,
dans lequel au moins une échelle de longueur est agencée en plus de la couche thermosensible pour déterminer la pointure de pied de l'utilisateur.

15. Agencement selon l'une quelconque des revendications 12 à 14 précédentes,
dans lequel la table de consultation est un tirage et les catégories dans les champs sont représentées par différents symboles.

16. Agencement selon l'une quelconque des revendications 12 à 15 précédentes, dans lequel
a. chaque catégorie de type de pied est représentée par une couleur différente,
b. chaque catégorie d'axe de jambe est représentée par un symbole géométrique différent, et
c. chaque type de structure de semelle intérieure est représenté par un symbole géométrique coloré, dont la couleur correspond à la catégorie de type de pied respective et le symbole géométrique correspond à la catégorie d'axe de jambe respective, laquelle combinaison est combinée par l'opérateur logique au type de structure de semelle intérieure respectif.

17. Agencement selon l'une quelconque des revendications 12 à 16 précédentes, dans lequel la table de consultation comprend un troisième champ (60) avec au moins deux catégories de type de structure de semelle intérieure.

18. Agencement selon l'une quelconque des revendications 12 à 17 précédentes, dans lequel
a. le premier champ (40) comprend une catégorie de pied plat, de pied à voûte plantaire faiblement cambrée, de pied neutre et de pied à voûte plantaire très cambrée,
b. le deuxième champ (50) comprend une catégorie de jambe arquée, de jambe neutre et de genoux cagneux, et/ou
c. le troisième champ (60) comprend une catégorie de support de pied latéral, neutre et interne.

19. Produit de programme informatique, qui est adapté pour réaliser les étapes suivantes lorsqu'il est exécuté sur un ordinateur :
a. réception d'une catégorie de type de pied en tant que première entrée,
b. réception d'une catégorie de type d'axe de jambe en tant que seconde entrée, ledit axe de jambe étant défini par la distance entre les genoux de l'utilisateur et les articulations de cheville de l'utilisateur lorsqu'il se tient debout, dans lequel un utilisateur ayant un contact des surfaces de peau internes du genou et des surfaces de peau internes de l'articulation de la cheville lorsqu'il se tient debout est défini comme ayant un axe de jambe neutre, une distance entre les surfaces de peau internes du genou définit un axe de jambe arquée, et une distance entre les surfaces de peau internes de l'articulation de cheville définit un axe de genoux cagneux,
c. détermination d'une catégorie de type de structure de semelle intérieure basée sur la première et la seconde entrée, en utilisant des opérations logiques selon les revendications 8 à 10 précédentes, et
d. fourniture en sortie de la catégorie de type de structure de semelle intérieure déterminée à l'étape c).

20. Produit de programme informatique selon la revendication 19,
dans lequel l'étape a) comprend
- la réception de données de type de pied acquises par analyse thermique d'une face de dessous du pied d'un utilisateur,
- l'analyse des données de type de pied, et
- la détermination du type de pied de l'utilisateur en se basant sur les données à l'aide d'un traitement d'image.
